## Europäisches Patentamt
## European Patent Office
## Office européen des brevets

(19)

(11) Veröffentlichungsnummer: **0 006 408**
**B1**

(12) **EUROPÄISCHE PATENTSCHRIFT**

(45) Veröffentlichungstag der Patentschrift: **24.02.82**

(51) Int. Cl.³: **A 61 F 1/00, A 61 F 1/03**

(21) Anmeldenummer: **78101483.2**

(22) Anmeldetag: **30.11.78**

(54) In eine Markhöhle einsetzbares Hilfselement zur Sicherung des Sitzes eines Implantates.

(30) Priorität: **30.06.78 DE 7819583 U**

(43) Veröffentlichungstag der Anmeldung:
**09.01.80 Patentblatt 80/1**

(45) Bekanntmachung des Hinweises auf die Patenterteilung:
**24.02.82 Patentblatt 82/8**

(84) Benannte Vertragsstaaten:
**BE CH DE FR GB LU NL SE**

(56) Entgegenhaltungen:
**DE - A1 - 2 617 749**
**DE - B1 - 2 338 136**
**DE - U1 - 7 819 583**
**GB - A - 1 409 053**
**GB - A - 1 430 071**

(73) Patentinhaber: **Howmedica International, Inc.**
**Zweigniederlassung Kiel**
**Professor-Küntscher-Strasse 1-5**
**D-2301 Schönkirchen üb. Kiel (DE)**

(72) Erfinder: **Seidel, Hartmut, Dr. med.**
**Hochrad 19**
**D-2000 Hamburg 52 (DE)**
Erfinder: **Richter, Karl M., Dipl.-Ing.**
**Haferkamp 14**
**D-2304 Wendtorf (DE)**
Erfinder: **Harder, Hans Erich**
**Mecklenburger Strasse 32**
**D-2301 Probsteierhagen (DE)**
Erfinder: **Behrens, Klaus, Ing. grad.**
**Am Sportplatz 8**
**D-2351 Rickling (DE)**

(74) Vertreter: **Hauck, Hans, Dipl.-Ing. et al,**
**Mozartstrasse 23**
**D-8000 München 2 (DE)**

Courier Press, Leamington Spa, England.

In eine Markhöhle einsetzbares Hilfselement zur Sicherung des Sitzes eines Implantates.

Die Erfindung bezieht sich auf ein in eine Markhöhle einsetzbares Hilfselement zur Sicherung des Sitzes von Implantaten.

Implantate werden häufig mit Hilfe eines Knochenzementes in der Markhöhle fixiert. Es besteht jedoch Gefahr, daß nach einem mehr oder weniger langen Zeitraum sich das Implantat unter der ständigen Beanspruchung lockert. Dies wird zum Teil dadurch verursacht, daß das Implantat nicht wirksam genug im Knochenzement eingebettet ist. Denn durch das Einsetzen des Implantates besteht die Gefahr, daß das Zement in Längsrichtung des Knochens auswandert, so daß ein Teil des Konchenzementes zur Fixierung des Implantates nicht beiträgt.

Es ist ein in einen Knochen einsetzbarer Köcher zur Aufnahme des Schaftes eines Implantates bekannt, der an der Außenseite Verankerungsmittel zur Fixierung in der Markhöhle aufweist und an den dem Schaft des Implantates abgewandten Ende geschlitzt ist (DE—B1—2 338 136). Der Köcher besitzt einen Hohlraum für die Aufnahme eines Spreizkörpers, mit dem der Köcher ohne Verwendung von Knochenzement fest in dem Knochen verankert werden kann.

Der Erfindung liegt die Aufgabe zugrunde, ein Hilfselement zur Sicherung des Sitzes von Implantaten anzugeben, das eine innige Verbindung zwischen Implantat und Knochenzement einerseits und Knochenzement und Knochen andererseits gewährleistet.

Diese Aufgabe wird erfindungsgemäß durch einen in die Knochenmarkhöhle einsetzbaren und das Hilfselement bildenden Stopfen der die zur Fixierung des Implantates nötige Menge Knochenzement zurückhält, und der einen vorderen glatten Abschnitt und einen hinteren flexiblen Anschnitt aufweist, der größere Querabmessungen als der vordere Abschnitt hat und mit Verankerungsmitteln zum Verankern des Stopfens in der Markhöhle versehen ist.

Der glatte Abschnitt hat in etwa den Durchmesser der Markhöhle, während der hintere etwas größeren Durchmesser besitzt.

Der Stopfen nach der Erfindung wird als erstes in die Knochenmarkhöhle eingeführt, bevor der Knochenzement eingegeben wird. Das einzusetzende Implantat preßt dann den Knochenzement seitlich in den spongiösen Teil des Knochens, wobei der Stopfen das Auswandern des Zements in Längsrichtung des Knochens verhindert. Dadurch entsteht zwischen Knochen und Implantat mit Hilfe des Zements eine feste, porenfreie Verbindung, wodurch die Haftung des Implantats wesentlich erhöht wird.

Eine besonders wirksame Verankerung des Stopfens in der Markhöhle wird mit einer weiteren Ausgestaltung der Erfindung erzielt, bei der die Verankerungsmittel von sägezahnartig umlaufenden Rippen gebildet sind. Die Sägezahnform der Rippen ist jedoch so angeordnet, daß der größere widerhakenförmige Widerstand in Einsetzrichtung des Stopfens stattfindet, während er in entgegengesetzter Richtung verhältnismäßig leicht herausgezogen werden kann. Hieran läß sich besonders leicht der Unterschied zwischen dem Hilfselement nach der Erfindung und dem oben beschriebenen bekannten Köcher erkennen. Die Verankerungsmittel am Köcher sollen in erster Linie gewährleisten, daß der Köcher nicht aus der Markhöhle herausgezogen wird, während in entgegengesetzter Richtung ein Flansch ein weiteres Einwandern des Köchers in den Knochen verhindert. Es sei an dieser Stelle auch angemerkt, daß der erfindungsgemäße Stopfen von dem Implantat völlig getrennt ist und bei der Anwendung ein mehr oder weniger großer Abstand zwischen dem Stopfen und dem freien Ende des Implantatenschaftes besteht. Während beim bekannten Köcher dieser den Implantate-schaft unmittelbar aufnimmt und lagert, dient bei der Erfindung der Stopfen dazu, ein Wandern des Knochenzements in Richtung der Markhöhle zu begrenzen.

Nach einer weiteren Ausgestaltung der Erfindung ist vorgesehen, daß der hintere Abschnitt sich nach hinten konisch erweitert. Zur Verbesserung der Flexibilität des hinteren Abschnitts ist erfindungsgemäß vorgesehen, daß die Wand des hohlen hinteren Abschnitts mindestens einen Längsschlitz aufweist. Nach einer weiteren Ausgestaltung der Erfindung ist im vorderen massiven Abschnitt des Stopfens eine Gewindebohrung vorgesehen. Mit der Gewindebohrung kann ein Einsatzwerkzeug in Eingriff kommen, um den Stopfen in gewünschter Tiefe in der Markhöhle zu verankern.

Nach einer weiteren Ausgestaltung der Erfindung ist im vorderen massiven Abschnitt eine Durchgangsbohrung vorgesehen. Die Durchgangsbohrung dient dazu, zu verhindern, daß komprimiertes Medium beim Einsetzen des Stopfens über die Durchgangsbohrung entweichen kann. Die Durchgangsbohrung ist jedoch vorzugsweise so eng gehalten, daß Knochenzement in nennenswertem Maße nicht hindurchtreten kann.

Ein Ausführungsbeispiel der Erfindung wird nachfolgend anhand von Zeichnungen näher beschrieben.

Fig. 1 zeigt die Längsansicht eines Stopfens nach der Erfindung.

Fig. 2 zeigt einen Schnitt durch den Stopfen nach Fig. 1.

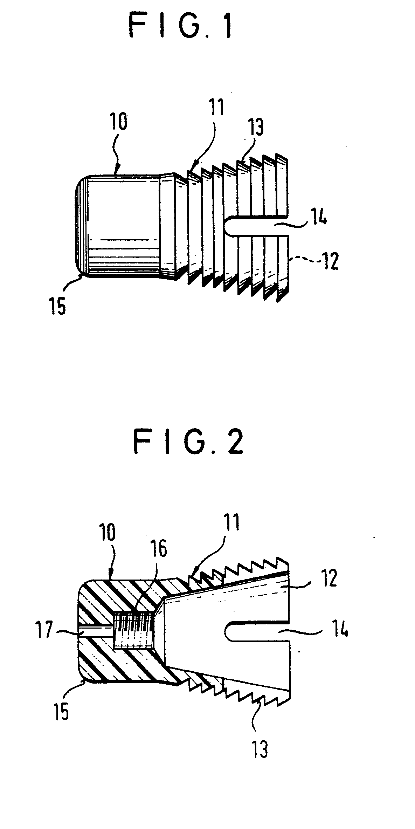

Der in den Figuren 1 und 2 gezeigte Stopfen, beispielsweise aus Polyethylen, besitzt einen vorderen, aus vollem Material hergestellten Abschnitt 10 und einen hinteren Abschnitt 11. Der hintere Abschnitt 11 ist kegelstumpfförmig gestaltet mit einer konischen Blindbohrung 12 im Inneren und sägezahnförmigen umlaufenden Rippen 13 außen. Außerdem erstrecken sich in Längsrichtung vier um 90° Umfangsabstand beabstandete Längsschlitze 14 durch die Wand des hinteren Abschnitts 11.

Die Flanken zwischen den Rippen bilden einen Winkel von etwa 60°. Sie sind derart angeordnet, daß eine widerhakenartige Wirkung entsteht, wenn der Stopfen in Einsetzrichtung, d.h. mit dem vorderen Abschnitt 10 zuerst in eine Knochenhöhle eingeführt wird. In entgegengesetzter Richtung ermöglichen die oberen Flanken der Rippen 13 ein verhältnismäßig leichtgängiges Gleiten.

Beim Einsetzen des Stopfens nach den Figuren 1 und 2 in eine Markhöhle ist darauf zu achten, daß der Außendurchmesser des vorderen Abschnitts 10, der an der vorderen Stirnseite mit einer abgerundeten Kante 15 versehen ist, dem Durchmesser der Markhöhle entspricht. Demzufolge werden die vier durch die Schlitze 14 getrennten Abschnitte des hinteren Abschnitts radial nach innen gebogen, wobei die sägezahnartigen Rippen 13 wirksam in der Markhöhle verankert werden, so daß beim Einsetzen eines Implantates der Knochenzement an einem Auswandern in Längsrichtung des Knochens gehindert ist und seitlich in den spongiösen Teil des Knochens gepreßt wird.

Bei eventuellen Reimplantationen wird der gezeigte Stopfen mit einem Spiralbohrer oder dergleichen durchbohrt. Mit einem Extraktionshaken, der durch das gebohrte Loch das Ende des Stopfens erfaßt, kann dieser herausgeschlagen werden.

Die Bohrung 12 setzt sich im massiven Abschnitt 10 in einer Gewindebohrung 16 fort, mit welcher ein Einsetzgerät in Eingriff gebracht werden kann. Zusätzlich oder alternativ kann auch die Bohrung 12 mit Innengewinde versehen werden, z.B. um den Stopfen zu extrahieren. Die Bohrung 16 hat gegenüber der Bohrung 12 einen reduzierten Durchmesser. Eine Durchgangsbohrung 17 im massiven vorderen Abschnitt von noch geringerem Durchmesser dient beim Einsetzen des Stopfens in die Markhöhle zum Druckausgleich.

Der gezeigte Stopfen hat Originalgröße für einen Durchmesser 12 mm. Normalerweise reicht eine Serie vom Innendurchmesser 10 bis 18 mm aus, um allen anfallenden Versorgungsfällen gerecht zu werden.

In den Zeichnungen ist ein Stopfen mit einem massiven vorderen und einem hohlen flexiblen hinteren Teil dargestellt. Die Erfindung ist jedoch hierauf nicht beschränkt. Vielmehr erstreckt sich die Erfindung auf alle stopfenartigen Elemente, welche, in eine Markhöhle eingesetzt, eine Wanderung von Knochenzement in Längsrichtung des Knochens verhindern, indem das stopfenartige Element dem einen Widerstand entgegensetzt.

**Patentansprüche**

1. In eine Markhöhle einsetzbares Hilfselement zur Sicherung des Sitzes von Implantaten, gekennzeichnet durch einen in die Knochenmarkhöhle einsetzbaren und das Hilfselement bildenden Stopfen, der die zur Fixierung des Implantates nötige Meng Knochenzement zurückhält, und der einen vorderen glatten Abschnitt (10) und einen hinteren flexiblen Abschnitt (11) aufweist, der größere Querabmessungen als der vordere Abschnitt (10) hat und mit Verankerungsmitteln (13) zum Verankern des Stopfens in der Markhöhle versehen ist.

2. Hilfselement nach Anspruch 1, dadurch gekennzeichnet, daß die Verankerungsmittel von sägezahnartig umlaufenden Rippen (13) gebildet sind.

3. Hilfselement nach Anspruch 1 oder 2, dadurch gekennzeichnet daß der hintere Abschnitt (11) sich nach hinten konisch erweitert.

4. Hilfselement nach einem der Ansprüche 1 bis 3, dadurch gekennzeichnet, daß die Wand des hohlen hinteren Abschnitts (11) mindestens einen Längsschlitz (14) aufweist.

5. Hilfselement nach einem der Ansprüche 1 bis 4, dadurch gekennzeichnet, daß im vorderen massiven Abschnitt (10) eine Gewindebohrung (16) vorgesehen ist.

6. Hilfselement nach einem der Ansprüche 1 bis 5, dadurch gekennzeichnet, daß in vorderen massiven Abschnitt (10) eine Durchgangsbohrung (17) vorgesehen ist.

**Claims**

1. An auxiliary element adapted to be inserted into a marrow cavity for securing the setting of implant, characterized by a stopper adapted to be inserted into the cavity of the marrow of bone and forming the auxiliary element, said stopper retaining the amount of bone cement necessary to fix the implant and comprising a smooth forward portion (10) and a flexible rearward portion (11), said rearward portion having transverse dimensions greater than those of the forward portion (10) and being provided with anchoring means (13) for anchoring the stopper in the marrow cavity.

2. An auxiliary element according to claim 1, characterized in that the anchoring means are formed of circumferentially extending saw-tooth shaped ribs (13).

3. An auxiliary element according to claim 1 or 2, characterized in that the rearward portion (11) is conically enlarging rearwardly.

4. An auxiliary element according to any one of the claims 1 to 3, characterized in that the wall of the hollow rearward portion (11) comprising at least one elongated slot (14).

5. An auxiliary element according to any one of the claims 1 to 4, characterized in that a threaded bore (16) is provided in the solid forward portion (10).

6. An auxiliary element according to any one of the claims 1 to 5, characterized in that a throughbore (17) is provided in the solid forward portion (10).

## Revendications

1. Elément auxiliaire pouvant être inséré dans une cavité médullaire pour assurer l'assise d'implants, caractérisé par un bouchon qui peut être inséré dans la cavité médullaire de l'os, et qui forme l'élément auxiliaire, ce bouchon retenant la quantité de ciment pour os qui est nécessaire à la fixation de l'implant, et présentant une partie antérieure lisse (10) et une partie postérieure flexible (11), qui a de plus grandes dimensions transversales que la partie antérieure (10), et qui est munie de moyens d'ancrage (13) pour l'ancrage du bouchon dans la cavité médullaire.

2. Elément auxiliaire selon la revendication 1, caractérisé par le fait que les moyens d'ancrage sont formés de nervures circonférentielles en dents de scie (13).

3. Elément auxiliaire selon la revendication 1 ou 2, caractérisé par le fait que la partie postérieure (11) s'élargit coniquement vers l'arrière.

4. Elément auxiliaire selon l'une des revendications 1 à 3, caractérisé par le fait que la paroi de la partie postérieure creuse (11) présente au moins une fente longitudinale (14).

5. Elément selon l'une quelconque des revendications 1 à 4, caractérisé en ce qu'un perçage taraudé (16) est prévu dans la partie antérieure massive (10).

6. Elément selon l'une quelconque des revendications 1 à 5, caractérisé en ce qu'un perçage traversant (17) est prévu dans la partie antérieure massive (10).

# F I G. 1

# F I G. 2